# EUROPÄISCHE PATENTANMELDUNG

(11) **EP 2 364 714 A1**
(43) Veröffentlichungstag der Anmeldung: **14.09.2011**
(21) Anmeldenummer: 10189112.5
(22) Anmeldetag: 27.10.2010
(51) Int. Cl.: A61K 36/28, A61K 36/76, A61K 36/38, A61K 36/53, A61K 36/77

(54) **Schmerzlindernde pflanzliche Zusammensetzung zum Auftragen auf die Haut**

(30) Priorität: 28.10.2009 DE 102009051066
(71) Anmelder: Stockinger-Berner, Petra, 48619 Heek (DE)
(72) Erfinder: Berner, Udo, 48739 Lechten (DE)
(74) Vertreter: Albrecht, Ralf

(57) **Zusammenfassung**

Die Erfindung betrifft einen thermochemischen Wärmespeicher (1, 22) zur Aufnahme, Umwandlung, Speicherung und Abgabe von Reaktionswärme durch reversible Umsetzung eines ersten partikulären Feststoffs (13) zu einem zweiten partikulären Feststoff (14) und einem Reaktionsfluid (19), wobei der Wärmespeicher (1, 22) wenigstens einen Reaktionsraum (2), eine hieran angeschlossene Reaktionsfluidleitung (7, 29) und wenigstens einen Wärmetauscher (9) aufweist, über den mittels einer externen Energiequelle oder eines Verbrauchers Energie zu- oder abgeführt werden kann, welcher dadurch gekennzeichnet ist, dass an den wenigstens einen Reaktionsraum (2) über Feststoffleitungen (3, 4) zwei Feststoffspeicher (5, 6) zur jeweiligen Speicherung der partikulären Feststoffe (13, 14) angeschlossen sind und zumindest eine Feststofffördereinrichtung (15) vorgesehen ist, um die partikulären Feststoffe (13, 14) zwischen Reaktionsraum (2) und den Feststoffspeichern (5, 6) unter Ausbildung eines Partikelstroms im Reaktionsraum (2) zu fördern.

## Beschreibung

Die vorliegende Erfindung betrifft eine Zusammensetzung zum Auftragen auf die Haut, insbesondere zur Linderung von Glieder-, Sehnen- und Muskelschmerzen sowie zur Schmerzlinderung bei Rheumaerkrankungen, insbesondere bei Weichteilrheumaerkrankungen.

Aus dem Stand der Technik ist eine Vielzahl von unterschiedlichen Salben, Lotionen, Tinkturen und ähnlichen Zusammensetzungen zur äußerlicher Anwendung bekannt, welche zur Linderung der oben genannten Schmerzen eingesetzt werden. Viele dieser Zusammensetzungen, insbesondere Sportsalben und sogenannte Pferdesalben enthalten als Hauptbestandteil Menthol, welches zwar eine stark kühlende Wirkung besitzt, jedoch letztlich keine nachhaltige Linderung der Schmerzen bewirkt.

Daneben gibt es eine Reihe von Salben, die durch medizinische Wirkstoffe eine gesteigerte schmerzlindernde Wirkung besitzen. Bekannte Wirkstoffe sind beispielsweise Natrium-Diclofenac, das u.a. in Voltaren®-Salbe zur Anwendung kommt oder auch Heparin-Natrium, welches ebenfalls in schmerzlindernden Salben verwendet wird. Solche medizinischen Salben besitzen zwar aufgrund der schmerzlindernden und entzündungshemmenden Wirkstoffe eine gute Wirksamkeit, haben jedoch den Nachteil, dass es zu Unverträglichkeitsreaktionen gegenüber diesen Wirkstoffen kommen kann. Diese können in einer Überempfindlichkeit gegenüber diesen Substanzen begründet sein oder sich in Nebenwirkungserscheinungen wie Hautrötungen etc. äußern. So ist es beispielsweise bekannt, dass Diclofenac zur Störung bei Blutbildung führen und darüber hinaus eine Überempfindlichkeit der Haut gegen Sonnenlicht an den behandelten Stellen auftreten kann. Zudem wird Diclofenac eine Herzinfarkt-fördernde Wirkung bestätigt, so dass auch bei äußerlicher Anwendung bei Herzinfarkt-gefährdeten Personen von der Behandlung mit Diclofenac-haltigen Salben Abstand genommen werden sollte.

Die Aufgabe der vorliegenden Erfindung besteht somit in der Schaffung einer Zusammensetzung zur äußerlichen Anwendung zur Linderung von Glieder-, Sehnen- und Muskelschmerzen sowie zur Schmerzlinderung bei Rheumaerkrankungen, insbesondere bei Weichteilrheumaerkrankungen, die einerseits eine gute schmerzlindernde Wirkung aufweist und dabei andererseits ohne die nachteiligen Nebenwirkungen der oben genannten Schmerzbehandlungsmittel auskommt.

Die Aufgabe wird bei einer Zusammensetzung der eingangs genannten Art dadurch gelöst, dass diese die folgenden Bestandteile enthält:
ein Trägermedium
   0,1 bis 1,0 Gew.-% Menthol
   1 bis 5 Gew.-% Rosskastaniensamenextrakt
   1 bis 5 Gew.-% Silberweidenrindenextrakt,
   2 bis 8 Gew.-% Arnicatinktur
   0,25 bis 5 Gew.-% Rosmarinöl
   1 bis 5 Gew.-% Johanniskrautöl.

Ein weiterer Gegenstand der vorliegenden Erfindung betrifft die Verwendung einer erfindungsgemäßen Zusammensetzung zur Herstellung eines Arzneimittels zur insbesondere äußerlichen Behandlung von Glieder-, Sehnen-, Muskelschmerzen und Rheumaerkrankungen, insbesondere Weichteilrheumaerkrankungen bei Menschen und Tieren.

Der vorliegenden Erfindung liegt die Erkenntnis zugrunde, dass durch die Kombination dieser natürlichen Wirkstoffe einerseits eine gute schmerzlindernde Wirkung in Bezug auf die oben genannten Schmerzen erzielt wird, ohne dass es zu den zuvor genannten Nebenwirkungen wie bei den pharmazeutischen Wirkstoffen kommt. Insbesondere hat sich herausgestellt, dass die Verwendung von Silberweidenrindenextrakt in Kombination mit den weiteren Bestandteilen sich besonders vorteilhaft auf die dauerhafte Schmerzlinderung auswirkt. So haben bei Probandenstudien mit dreißig Personen 80% der Befragten angegeben, bereits nach einer Behandlung von drei bis fünf Tagen eine deutliche Minderung des Schmerzempfindens verspürt zu haben.

Als im Rahmen der erfindungsgemäßen Zusammensetzungen einsetzbare Trägermedien kommen prinzipiell sämtliche für diese Zwecke geeigneten und bekannten Zusammensetzungen in Frage. Diese können hydrophil bzw. wasserbasiert sein, wozu in der Regel ein Verdicker eingesetzt wird, um die gewünschte Viskosität bzw. Verstreichbarkeit zu erzielen. Auch lipophile Trägermedien können verwendet werden, so zum Beispiel paraffinwachshaltige Salbengrundlagen, wie Vaseline.

Ebenso kann das Trägermedium auch eine w/o oder eine o/w Emulsion sein.

Je nach Wahl des Trägermediums kann die erfindungsgemäße Zusammensetzung eine Emulsion sein, wie beispielsweise eine Creme, eine Lotion oder eine Milch. Die Zusammensetzung kann weiter als Salbe, Gel, Öl, Balsam oder Serum gefertigt sein.

Gemäß einer Weiterbildung der erfindungsgemäßen Zusammensetzung enthält diese 0,15 bis 0,5 Gew.-%, bevorzugt 0,25 Gew.-% Menthol. Ein Mentholgehalt in dieser Größenordnung ist vorteilhaft, da hierdurch einerseits eine angenehm kühlende Wirkung auf der Haut erzielt wird, so dass bereits kurz nach dem Auftragen der Salbe das Gefühl einer Schmerzlinderung erreicht wird. Außerdem wird gleichzeitig eine Durchblutungsförderung der Haut erreicht, wodurch sich eine antiinflammatorische Wirkung einstellt. Insofern dient der Mentholbestandteil in der erfindungsgemäßen Zusammensetzung dazu, eine unmittelbar schmerzlindernde Wirkung zu erzielen, wohingegen die übrigen Bestandteile prinzipiell eine mittel- bis langfristige Linderung der Schmerzen bewirken können.

Weiterhin kann die erfindungsgemäße Zusammensetzung 2,0 bis 3,0 Gew.-%, bevorzugt 2,5 Gew.-% Rosskastaniensamenextrakt enthalten. Diese Menge hat sich als besonders vorteilhaft erwiesen, da in Kombination mit den übrigen Bestandteilen der Zusammensetzung eine gute schmerzlindernde Wirkung bei gleichzeitiger Minimierung von Überempfindlichkeitsreaktionen erzielbar ist.

Aus demselben Grunde hat es sich ebenfalls als vorteilhaft herausgestellt, wenn die erfindungsgemäße Zusammensetzung 2,0 bis 3,0 Gew.-%, insbesondere 2,5 Gew.-% Silberweidenrindenextrakt enthält. Wie bereits eingangs ausgeführt haben sich die erfindungsgemäßen Salben bzw. Zusammensetzungen durch Kombination von Silberweidenrindenextrakt mit den übrigen Pflanzenextrakten als besonders wirksam bei der Schmerzbekämpfung gezeigt.

Weiterhin bevorzugt ist es, wenn die Zusammensetzung 3,0 bis 6,0 Gew.-%, insbesondere 5,0 Gew.-% Arnicatinktur enthält. Bei einem solchen Gehalt wird eine gute Wirksamkeit bei gleichzeitig geringem hautreizenden Potential erreicht.

Weiterhin bevorzugt aus den oben genannten Gründen ist eine Zusammensetzung, die 0,5 bis 2,0 Gew.-%, bevorzugt 0,75 Gew.-% Rosmarinöl enthält.

Schließlich hat es sich als vorteilhaft herausgestellt, wenn die erfindungsgemäße Zusammensetzung mit den oben genannten Bestandteilen 2,0 bis 3,0 Gew.-%, insbesondere 2,5 Gew.-% Johanniskrautöl enthält.

Die genannten Wirkstoffe, d.h. Menthol, Rosskastaniensamenextrakt, Silberweidenrindenextrakt, Arnicatinktur, Rosmarinöl und Johanniskrautöl verstehen sich bezüglich ihrer Einsatzmengen als reine Naturstoffe, d.h. bezogen auf das jeweils unverdünnte Extrakt. Selbstverständlich ist es möglich, die genannten Naturstoffe auch in einem Lösungsmittel, insbesondere in alkoholischer, ölhaltiger oder wässriger Lösung bzw. Tinktur bei der Zubereitung der erfindungsgemäßen Zusammensetzung einzusetzen.

Nach einer Weiterbildung der erfindungsgemäßen Zusammensetzung weist diese ein wässriges Trägermedium auf, welches einen oder mehrere Polyacrylsäure-basierten Verdicker enthält. Solche Trägermedien sind zeichnen sich gegenüber ölhaltigen Trägermedien dadurch aus, dass sie schneller auf der Haut trocknen bzw. einziehen und kein fettiges Hautgefühl hinterlassen.

Besonders bevorzugt kann der Polyacrylsäure-basierte Verdicker eine Viskosität von 50000 mPas besitzen. Solche Polyacrylsäure-basierten Verdicker sind beispielsweise unter dem Handelsnamen Carbomer® 50000 erhältlich.

Erfindungsgemäße Zusammensetzungen weisen einen Gehalt von insbesondere 0,25 bis 5 Gew.-%, besonders bevorzugt 0,75 Gew.-% an den zuvor genannten Verdickern auf. Solche Einsatzmengen erlauben die einfache Einstellung der gewünschten Konsistenz der Zusammensetzung. Hierzu wird in Wasser eine entsprechende Menge des Verdickers eingerührt, wobei die Zusammensetzung gleichzeitig auch leicht erwärmt werden kann.

Gemäß einer bevorzugten Ausführungsform der erfindungsgemäßen Zusammensetzung enthält diesen eine oder mehrere Alkohole, die ausgewählt sind aus aliphatischen Alkoholen mit zwei bis acht Kohlenstoffatomen zu den Mischungen dieser Alkohole. Hierbei kommen insbesondere primäre und sekundäre Mono-Alkohole in Betracht, wie beispielsweise Ethanol, 1-Propanol und/oder 2-Propanol. Darüber hinaus können ebenfalls Alkohole mit mehr als zwei Hydroxi-Gruppen verwendet werden wie Glycerin. Bevorzugte erfindungsgemäße Zusammensetzungen weisen einen Gehalt von 0,5 bis 2 Gew.-% Ethanol und 10 bis 30 Gew.-% 2-Propanol, besonders bevorzugt 1 Gew.-% Ethanol und 20 Gew.-% 2-Propanol auf.

Erfindungsgemäße Zusammensetzungen können darüber hinaus einen oder mehrere pH-Regulatoren enthalten. Vorzugsweise werden hierbei alkalisch wirkende Verbindungen, insbesondere Alkali- und Erdalkalicarbonate sowie Alkali- und Erdalkalihydroxyde verwendet. Besonders bevorzugt ist der Einsatz von Natrium- oder Kaliumhydroxyd, wobei eine bevorzugte Zusammensetzung 0,5 bis 2 Gew.-%, insbesondere 1,5 Gew.-% an Natrium- oder Kaliumhydroxyd enthält.

Außerdem können auch organische oder anorganische Säuren und/ oder deren Salze als pH-Regulatoren verwendet werden wie beispielsweise Phosphorsäure, a-Hydroxycarbonsäuren, vorteilhafterweise Fruchtsäuren, insbesondere Milchsäure, Citronensäure, Äpfelsäure und/oder Weinsäure, sowie ß-Hydroxycarbonsäuren, vorteilhafterweise Säuren mit zusätzlich konservierenden Charakter wie Ameisensäure, Salicylsäure, Essigsäure. sowie Sulfonsäure - und/oder deren Derivate oder Mischungen aus diesen zum Einsatz. Auch der Einsatz langkettiger Säuren ist möglich, wie beispielsweise Linolsäure, Linolensäure, 12- Hydroxystearinsäure, Stearinsäure, Isostearinsäure, Palmitinsäure, 11- Hydroxypalmitinsäure, Proteinhydrolysatkondensate (z. B. Palmitoylglutaminsäure), Hyaluronsäure.

Auch Aminosäuren können verwendet werden, wie z.B. Arginin, Alanin, Histidin, Tyrosin, Phenylalanin, DOPA (Dihydroxyphenylalanin), Ornithin, Lysin, Taurin und Tryptophan, Prolin, Asparaginsäure, Carnetin, Cystein, Glutamin, Glycin, Isoleucin, Leucin, Methionin, Serin, Threonin, Tryptophan, Valin, Asparagin, Glutaminsäure, insbesondere Glutathion, Cystein und/oder Taurin.

Erfindungsgemäße Zusammensetzungen können darüber hinaus weitere Inhaltsstoffe aufweisen. Hierfür kommen beispielsweise einzeln oder in beliebiger Mischung in Frage:
Pflanzen(samen)-Extrakte oder -pulver, vorzugsweise Extrakte aus Früchten, Wurzeln, Rinden und/oder Samen, wie z.B. Litschi(samen)-Extrakt, Traubenkernextrakte, Citrusfruchtextrakt, vorzugsweise Grapefruit(kern)-Extrakt, Acerola-Extrakt, Aronia-Extrakt, Melonen-Extrakt, Broccoli-Pulver, Broccolisprossen-Pulver oder - Extrakt, Tomaten-Extrakt, Ölpalmenfrucht-Extrakt, Bockshornklee-Extrakt, Kaffeebohnen-Extrakt, Leinsaat-Extrakt, Studentenblumen-Extrakt, Maca Wurzel-Pulver oder - Extrakt, Feigenkaktus(frucht- oder samen)-Extrakt, Oliven-Extrakt, Olivenblätter-Extrakt, Papaya-Extrakt, Dattel-Extrakt, Chicoree-Extrakt, Artischocken-Extrakt, Bananen-Extrakt, Pflaumen-Extrakt, Kirsch-Extrakt, Erdbeer-Extrakt, Extrakte der Centella Asiatica, Perilla(samen)-Extrakt, Reiskeim-Extrakt, Rooibostee-Extrakt, Sojakeimlinge-Extrakt, Traubenschalen-Extrakt, Trauben-Extrakt, Zimt-rinden-Extrakt, Apfel(samen)-Extrakt, Acker-/Odermenning-Extrakt, Bärentraubenblatt-Extrakt, Birkenblatt-Extrakt, Algen-Extrakt, Brunnenkresse-Extrakt, Calendula-Extrakt, Cinchona-Extrakt, Efeublatt-Extrakt, E-chinaceawurzel-Extrakt, Eibischwurzel-Extrakt, Enzianwurzel-Extrakt, Eukalyptusblatt-Extrakt, Geißblatt-Extrakt, Gingkoblatt-Extrakt, Ginsengwurzel-Extrakt, Große Klette Blatt-Extrakt, Guarana Samen-Extrakt, Gurken-Extrakt, Sellerie-Extrakt, Hafer-Extrakt, Hagedorn(Blüte)-Extrakt, Heartsease(Blüte)-Extrakt, Heckenrose(Frucht)-Extrakt, Henna(Blatt)-Extrakt, Hopfen-Extrakt, Jasmin(Blüte)-Extrakt, Kamille(Blüte)-Extrakt, Kapuzinerkresse-Extrakt, Karotten-Extrakt, Knolliges Mädesüß-Extrakt, Kornblume-Ex-trakt, Lakritze(Wurzel)-Extrakt, Lavendel(Blüte)-Extrakt, Lilie(Blüte)-Extrakt, Melliot-Extrakt, Mohn(Blüte)-Extrakt, Mutterkraut(Blüte)-Extrakt, Spargel-Extrakt, Weißkohl-Extrakt, Grünkohl-Extrakt, Myrthe(Blüte)-Ex-trakt, Orchidess(Blüte)-Extrakt, Schafgarbe(Blüte)-Extrakt, Petersilie-Extrakt, Lauch-Extrakt, Pinie(Nadel)-Extrakt, Salbei(Blatt)-Extrakt, Schwarzwurz-Extrakt, Melonen-Extrakt, Zwiebel-Extrakt, Soapwort(Wurzel)-Extrakt, Stechpalme(Wurzel)-Extrakt, Thymian(Blatt)-Extrakt, Virginische Zaubernuß(Blatt)-Extrakt, Wacholder(Frucht)-Extrakt, Walnuss(Blatt)-Extrakt, Wasserlilie(Wurzel)-Extrakt, Weiße Nessel-Extrakt, Zinnkraut(Blatt)-Extrakt, Cystus-Extrakt (Cystus incanus ssp. Tauricus), Rosenrot (Rhodiola rosea)-Extrakt, Schisandra chinensis-Extrakt, Acanthopanax senticosus-Extrakt, Weihrauch-Extrakt, Gelbwurz-Extrakt, Granatapfel-Extrakt, Brennessel(Blatt)-Extrakt, Knoblauch-Extrakt, Heidelbeeren-Extrakt, Stachelbeeren-Extrakt, Blaubeeren-Extrakt, Himbeeren-Ex-trakt, Brombeeren-Extrakt, Johannisbeeren-Extrakt, Cranberry-Extrakt, Rote Beete-Extrakt, Salatpflanzen-Extrakt, Uncaria Tomentosa (Katzenkralle)-Extrakt, Rhabarber-Extrakt, Flieder-Extrakt, Holunderbeeren-Extrakt, Eichenrinde-Extrakt, Hamamelisblätter- und - rinde-Extrakt, Wassernabel-Extrakt, Beinwell-Extrakt, sowie Extrakte oder Pulver aus Nadelhölzern, Fichtennadeln, Latschenkiefer, Sumpfzypresse, Eiben, Samenfarne, Pfeilkraut, Drachenbaum, Schachblume, Gelbstern, Tulpe, Safran, Ananas, Zuckerrohr, Roggen, Mais, Wasserlinsen, Muskatnuß, Boldoblätter, Lorbeerblätter, Pfeffer, Schöllkraut, Erdrauchkraut, Rote Seifenwurzel, Bruchkraut, Gänsefußgewächse, Spinat, Rote Rübe, Vogelknöterichkraut, Birkengewächse, Stachelbeere, Steinbrechgewächse, Sonnentaugewächse, Hortensiengewächse, Mädesüßblüten, Blutwurzelstock, Erdbeerblätter, Hagebutten, Frauenmantelkraut, Weißdornblätter mit -blüten, Mirabelle, Aprikose, Pfirsich, Schlehe, Alexandriner-Sennes-Blätter, -Früchte, Tinnevelly-Sennes-Blätter, -Früchte, Hauhechelwurzel, Steinkleekraut, Bockshornkleesamen, Süßholzwurzel, Gartenbohne, Syzygii-cumini-Rinde, Affenbrotbaumrinde und -blätter, Eukalyptusblätter, Bitterorangenschale, Bitterholz, Mango, Senegawurzel, Ratanhiawurzel, Rosskastaniensamen, Spindelbaumgewächse, Faulbaumrinde, Kreudornfrüchte, Efeublätter, Taigawurzel, Zahnstocherammeifrüchte, Dillfrüchte, Engelswurzel, Kümmelfrüchte, Korianderfrüchte, Karotte, Fenchelfrüchte, Liebstöckelwurzel, Petersilienfrüchte, Anisfrüchte, Endivie, Cardui mariae herba, Stevia rebaudiana, Kolanüsse, Eberraute, Estragon, Gänseblümchen, Kürbis, Zucchini, Meerrettich, Radieschen, Feigen, Maniok-Knollen, Sanddornbeeren, Primula-Arten, Hortensie, Baldrianwurzel, Rhododendron-Arten, Schwalbenwurz, Harpagophyti radix, Plantaginis herba und/oder Indische Flohsamen.

Weiterhin können die Zusammensetzungen Proteine pflanzlichen Ursprungs enthalten, wie vorzugsweise Soja-, Mandel- , Erbsen-, Kakao-, Algen-, Kartoffel- und Weizenprotein, insbesondere aber Sojaproteine wie z.B. Vegeseryl® HGP (Sojaeiweiss Konzentrat; INCI: Glycine Soja (soybean) Protein) und/oder auch tierische Proteine, wie beispielsweise Elastin-, Kollagen-, Keratin-, Seiden- und Milcheiweiß-Protein.

Weiterhin können erfindungsgemäße Zusammensetzungen noch oberflächenaktive Substanzen, Antioxidantien und/oder hautpflegende Stoffe enthalten.

Die oberflächenaktiven Substanzen umfassen im wesentlichen zwei Gruppen, die Tenside und die Emulgatoren.

Im Rahmen der vorliegenden Erfindung werden unter dem Begriff Tenside grenzflächenaktive Substanzen verstanden, die an Ober- und Grenzflächen Adsorptionsschichten bilden oder in Volumenphasen zu Mizellkolloiden oder lyotropen Mesophasen aggregieren können. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Die im Rahmen der Erfindung einsetzbaren Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion.

Bezüglich der wahlweise eingesetzten Antioxidatien werden vorzugsweise natürliche oder naturidentische Antioxidantien eingesetzt, vorteilhafterweise in Mengen von 0,03 bis 8 Gew.-%, bevorzugt 0,05 bis 5 Gew.-%, und insbesondere 0,1 bis 1 Gew.-%, insbesondere ausgewählt aus terpenhaltigen Antioxidantien und/oder Vitamin E-(Derivaten), vorzugsweise in Kombination mit Vitamin C-(Derivaten). Vorzugsweise ist die Zubereitung frei von synthetischen Antioxidantien.

Zu den hautpflegenden Stoffen gehören insbesondere Feuchtigkeitsregulanzien, die vorzugsweise ausgewählt sind aus Polyolen, dabei vorzugsweise Glykol, Propylenglykol, Butylenglykol, Pentylenglykol, Hexylenglykol, Glycerin, Inositol, Sorbitol, Mannit, Palatinit, Maltodextrin, Dextrin, Cyclodextrin, Glucose, Fructose, Lactose, Mannose, Galaktose und/oder andere Saccharide, sowie aus Harnstoff, Aminosäuren und deren Salze, hier vorzugsweise Glutaminsäure, Glycin oder Glutamate, insbesondere Argininpyroglutamat, Lactat, Vitamin E, Lecithin, Glucoronsäure, Panthenol, Allantoin, Ceramide, Chondroitinsulfat, Natriumacrylat-Vinylalkohol-Co-polymere, Oligopeptide, 2-Pyrrolidon, Uronsäuren, Polysilicone, Arginine oder deren Gemische.

Die vorliegende Erfindung wird im Folgenden anhand eines Ausführungsbeispiels näher erläutert.

### Beispiel:

200g einer erfindungsgemäßen Salbe weisen die folgenden Inhaltsstoffe auf:

| | |
|---|---|
| 0,75 g | Carbomer® 50000 |
| 20,00 g | 2-Propanol |
| 1,50 g | Natriumhydroxid-Pellets |
| 1,00 g | Ethanol (90 Vol.-%) |
| 0,75 g | Menthol |
| 2,50 g | Rosskastaniensamenextrakt |
| 2,50 g | Silberweidenrindenextrakt |
| 5,00 g | Arnikatinktur |
| 0,75 g | Rosmarinöl |
| 2,50 g | Johanniskrautöl |
| ad 100 g | aqua pur. |

Zur Herstellung der erfindungsgemäßen Zusammensetzung wird zunächst das Wasser in einem Rührwerk vorgelegt, anschließend das Carbomer® 50000 hierin aufgelöst, dann Ethanol und Isopropanol hinzugegeben und schließlich das Natriumhydroxyd, welches in Form von Pellets eingesetzt wird, hierin aufgelöst. Diese Mischung wird zunächst intensiv gemischt und im weiteren Verlauf unter intensivem Rühren die natürlichen Pflanzenextrakte hinzugegeben, bis eine Salbe von gleichmäßiger Konsistenz erhalten wird.

## Patentansprüche

1. Zusammensetzung zum Auftragen auf die Haut, insbesondere zur Linderung von Glieder-, Sehnen- und Muskelschmerzen sowie zur Schmerzlinderung bei Rheumaerkrankungen, insbesondere bei Weichteilrheumaerkrankungen, enthaltend wenigstens die folgenden Bestandteile:
ein Trägermedium,
0,1 bis 1,0 Gew.-% Menthol,
1 bis 5 Gew.-% Rosskastaniensamenextrakt,
1 bis 5 Gew.-% Silberweidenrindenextrakt,
2 bis 8 Gew.-% Arnikatinktur,
0,25 bis 5 Gew.-% Rosmarinöl und
1 bis 5 Gew.-% Johanniskrautöl.

2. Zusammensetzung nach Anspruch 1, **dadurch gekennzeichnet, dass** die Zusammensetzung 0,15 bis 0,50 Gew.-%, insbesondere 0,25 Gew.-% Menthol enthält.

3. Zusammensetzung nach Anspruch 1 oder 2, **dadurch gekennzeichnet, dass** die Zusammensetzung 2,0 bis 3,0 Gew.-%, insbesondere 2,5 Gew.-% Rosskastaniensamenextrakt enthält.

4. Zusammensetzung nach einem der vorstehenden Ansprüche, **dadurch gekennzeichnet, dass** die Zusammensetzung 2,0 bis 3,0 Gew.-%, insbesondere 2,5 Gew.-% Silberweidenrindenextrakt enthält.

5. Zusammensetzung nach einem der vorstehenden Ansprüche, **dadurch gekennzeichnet, dass** die Zusammensetzung 3,0 bis 6,0 Gew.-%, insbesondere 5,0 Gew.-% Arnikatinktur enthält.

6. Zusammensetzung nach einem der vorstehenden Ansprüche, **dadurch gekennzeichnet, dass** die Zusammensetzung 0,5 bis 2,0 Gew.-%, insbesondere 0,75 Gew.-% Rosmarinöl enthält.

7. Zusammensetzung nach einem der vorstehenden Ansprüche, **dadurch gekennzeichnet, dass** die Zusammensetzung 2,0 bis 3,0 Gew.-%, insbesondere 2,5 Gew.-% Johanniskrautöl enthält.

8. Zusammensetzung nach einem der vorstehenden Ansprüche, **dadurch gekennzeichnet, dass** das Trägermedium wässig ist und einen oder mehrere polyacrylsäurebasierten Verdicker enthält, der insbesonder eine Viskosität von 50.000 mPas besitzt, wobei die Zusammensetzung insbesondere 0,25 bis 5 Gew.-%, vorzugsweise 0,75 Gew.-% dieses Verdickers enthält.

9. Zusammensetzung nach einem der vorstehenden Ansprüche, **dadurch gekennzeichnet, dass** die Zusammensetzung einen oder mehrere Alkohole enthält, die ausgewählt sind aus aliphatischen Alkoholen mit zwei bis acht Kohlenstoffatomen und Mischungen dieser, insbesondere Ethanol, 1-Propanol und/oder 2-Propanol, wobei die Zusammensetzung vorzugsweise 0,5 bis 2 Gew.-% Ethanol und 10 bis 30 Gew.-% 2-Propanol, besonders bevorzugt 1 Gew.-% Ethanol und 20 Gew.-% 2-Propanol aufweist.

10. Zusammensetzung nach einem der vorstehenden Ansprüche, **dadurch gekennzeichnet, dass** die Zusammensetzung einen oder mehrere pH-Regulatoren enthält, insbesondere Natriumhydroxid, wobei die Zusammensetzung 0,5 bis 2 Gew.-%, insbesondere 1,5 Gew.-% Natriumhydroxid enthält.

11. Verwendung einer Zusammensetzung nach einem der vorstehenden Ansprüche zur Herstellung eines Arzneimittels zur insbesondere äußerlichen Behandlung von Glieder-, Sehnen-, Muskelschmerzen und Rheumaerkrankungen, insbesondere Weichteilrheumaerkrankungen, bei Menschen und Tieren.
